# EUROPEAN PATENT APPLICATION

(11) **EP 3 808 262 A1**
(43) Date of publication of application: **21.04.2021**
(21) Application number: 20201728.1
(22) Date of filing: 14.10.2020
(51) Int. Cl.: A61B 5/042, A61B 5/053, A61B 5/00, A61B 5/0464

(54) **LOCAL RENDERING BASED MULTI-MODALITY SUBSET PRESENTATION**

(30) Priority: 15.10.2019 US 201916653433
(71) Applicant: Biosense Webster (Israel) Ltd., 2066717 Yokneam (IL)
(72) Inventor: ALTMANN, Andres Claudio, 2066717 Yokneam (IL); GLINER, Vadim, 2066717 Yokneam (IL); ZILBERMAN, Israel, 2066717 Yokneam (IL); PALTI, Yair, 2066717 Yokneam (IL); BETTAN, Remi, 2066717 Yokneam (IL)
(74) Representative: Small, Gary James

(57) **Abstract**

Methods, apparatus, and systems for medical procedures are disclosed herein and include receiving a first set of biometric data of a first modality for a first portion of a body part, determining first visual characteristics based on the first set of biometric data, receiving a second set of biometric data of a second modality for a second portion of the body part, the second portion of the body part being a subset of the first portion of the body part, and determining second visual characteristics based on the second set of biometric data. A first view including the first portion of the body part rendered with the first visual characteristics may be provided and second view comprising the second portion of the body part rendered with the second visual characteristics may be provided, the second view superimposed on the first view at a location on the first view corresponding to the second portion of the body part.

## Description

### FIELD OF INVENTION

The present application provides systems, apparatuses, and methods for improving intra-body visualization.

### BACKGROUND

Medical conditions such as cardiac arrhythmia (e.g., atrial fibrillation (AF)) are often diagnosed and treated via intra-body procedures. For example, electrical pulmonary vein isolation (PVI) from the left atrial (LA) body is performed using ablation for treating AF. PVI, and many other minimally invasive catheterizations, require real-time visualization and mapping of an intra-body surface.

Visualization and mapping of intra-body body parts can be performed by mapping propagation of activation waves, Fluoroscopies, computerized tomography (CT) and magnetic resonance imaging (MRI), as well as other techniques which may require a greater than desirable amount of time or resources to provide the visualization and mapping.

### SUMMARY

Methods, apparatus, and systems for medical procedures are disclosed herein and include receiving a first set of biometric data of a first modality for a first portion of a body part, determining first visual characteristics based on the first set of biometric data, receiving a second set of biometric data of a second modality for a second portion of the body part, the second portion of the body part being a subset of the first portion of the body part, and determining second visual characteristics based on the second set of biometric data. A first view including the first portion of the body part rendered with the first visual characteristics may be provided and second view comprising the second portion of the body part rendered with the second visual characteristics may be provided, the second view superimposed on the first view at a location on the first view corresponding to the second portion of the body part. The first modality may be a local activation time (LAT), an electrical activity, a topology, a bipolar mapping, a dominant frequency, or an impedance. The second modality may be a different one of an LAT, an electrical activity, a topology, a bipolar mapping, a dominant frequency, or an impedance, then the first modality. The body part may be a cardiac chamber. The first portion of the body part may be the entire body part or a subset of the body part.

A third view including a rendering based on the second set of biometric data may be provided.

The first visual characteristics may be based on a range of values within the first set of biometric data and the second visual characteristics may be based on a range of values within the second set of biometric data. The first modality or the second modality may be determined based on one of an automatic determination or a user input. The automatic determination may be based on one of a, system configuration, prior use, the first biometric data, the second biometric data, a patient history, and a stored setting.

A third set of biometric data of a third modality for the second portion of the body part may be determined. Third visual characteristics may be based on the third set of biometric data. The second view corresponding to the second portion of the body part may be rendered with the third visual characteristics. The second view may include the second portion of the body part rendered with the third visual characteristics and may be provided after the second view including the second portion of the body part is rendered with the second visual characteristics, instead of the third visual characteristics, is provided.

One or more catheters may be configured to sense the first set of biometric data of the first modality for a first portion of a body part and the second set of biometric data of the second modality for a second portion of the body part.

A display may be provided and may render the first view and the second view.

### BRIEF DESCRIPTION OF THE DRAWINGS

A more detailed understanding can be had from the following description, given by way of example in conjunction with the accompanying drawings wherein:
FIG. 1 is a diagram of an exemplary system in which one or more features of the disclosure subject matter can be implemented;
FIGS. 2 is a flowchart for displaying a global view with a first modality and a local view with a second modality;
FIG. 3 is an image of a local view superimposed on a global view;
FIG. 4A is a diagram of a first portion of a body part rendered as a global view and local view superimposed on the global view;
FIG. 4B is another diagram of the first portion of a body part of FIG. 4A rendered as a global view and different local view superimposed on the global view;
FIG. 4C is another diagram of the first portion of a body part of FIG. 4A rendered as a global view and two local views superimposed on the global view;
FIG. 5A is a diagram of a first portion of a body part rendered as a global view and selection of a local view;
FIG. 5B is a diagram of a first portion of a body part rendered as a global view and catheter location-based selection of a global view;
FIG. 6 is a diagram of a first portion of a body part rendered as a global view and a local view including a problem area; and
FIG. 7 is a diagram of a first portion of a body part rendered as a global view and local view superimposed on the global view with a third view corresponding to the local view.

### DETAILED DESCRIPTION

According to embodiments of the disclosed subject matter, a catheter or other insertable device may be inserted into a patient's body and may sense the biometric data of a patient's intra-body body part. For example, elements such as electrodes on an insertable catheter may sense electrical activity data on the surface of a cardiac chamber and provide the electrical activity data to a processor. The processor may generate rendering data that enables a display to render the shape of the cardiac chamber such that the surface of the cardiac chamber shows the electrical activity data using a visual characteristic, as further disclosed herein.

According to embodiments disclosed herein, one or more catheters may be used to collect biometric data of a body part for different modalities. A modality may be a category of biometric data and may include, for example, a LAT, an electrical activity, a topology, a bipolar mapping, a dominant frequency, or an impedance. The rendering of a body part may include biometric data for different modalities shown for different points of the body part. The biometric data may be rendered using visual characteristics (e.g., colors) shown on the surface of the body part via a display (e.g., a monitor). The biometric data may be visually conveyed using any applicable visual characteristic such as a range or gradient of colors, hues, saturations, patterns, shapes, protrusions (e.g., a 3D protrusion), textures, alphanumeric characters, or the like. To clarify, the shape of a body part, such as a heart, may be rendered via a display, and the surface of the shape may have a visual characteristics (e.g., colors) that convey the values of the biometric data (e.g., LAT values where a first color may represent a first lower range of LAT values and a second color may represent a second higher range of LAT values). The render of the body part may be adjustable such that the viewing angle, zoom amount, position, orientation, and other viewing properties may be adjusted by a user or automatically via a predetermined criteria or dynamically determined criteria.

According to embodiments of the disclosed subject matter, the rendering data may include a global view and a local view. The global view may include the biometric data for a first modality for a first portion of a body part, which may be the entire body part or the portion of the body part that is rendered on a display at a given time. The global view may show biometric data for the first modality on the surface of the first portion of the body part using visual characteristics such as colors that indicate different values of the biometric data (e.g., the modality may be LAT values, and high LAT values may be shown in red verses low LAT values may be shown in purple).

The visual characteristics that indicate different values of the biometric data for a first modality in the global view may be determined based on the range of values present in the first modality biometric data for first portion of the body part. A wider range of values may result in more values being indicated by the same or similar visual characteristics. For example, the first portion of the body part may be a cardiac chamber with LAT values that range from -500ms through +500ms. The visual characteristics used to indicate the LAT values in the global view may, for example, be five colors including red, yellow, green, blue, purple such that red may indicate -500ms through -301ms, yellow may indicate - 300ms through -101ms, green may indicate -100ms through +99ms, blue may indicate + 100ms through +299ms, and purple may indicate +300ms through +500ms.

The local view may be superimposed onto a portion of the global view and may include the biometric data for a second modality and for a second portion of the body part that is contained within the first portion of the body part shown in the global view (e.g., the first portion of the body part may be a cardiac chamber and the second portion of the body part may be a small portion of the cardiac chamber). The second modality for which the biometric data is displayed in the local view may be a different modality than the first modality for which the biometric data is displayed in the global view. For example, the global view may show LAT values for an entire cardiac chamber whereas the local view may show impedance values for a smaller subset of the cardiac chamber.

The local view may show biometric data of the second modality on the surface of the second portion of the body part using visual characteristics such as colors that indicate values of the second modality's biometric data (e.g., high impedance values may be shown in red vs low impedance values may be shown in purple). The visual characteristics that indicate different values of the second modality's biometric data for the local view may be determined based on the range of values present in the second modality's biometric data for the second portion of the body part, such that the range of values and corresponding visual characteristics are specific to the second, smaller, area of the body part. For example, the local view may show impendence values and may be superimposed on a global view that shows LAT values. The global view may show a cardiac chamber with LAT values that range from -500ms and +500ms indicated by colors and the local view, superimposed onto a portion of the global view over a smaller portion of the cardiac chamber may show impedance values that range from 2.2 ohms to 3.2 ohms. Accordingly, the visual characteristics used to indicate the impedance values within the local view may be the same five colors as the global view including red, yellow, green, blue, purple such that red may indicate 2.2 ohms to 2.4 ohms, yellow may indicate 2.4 ohms through 2.6 ohms, green may indicate 2.6 ohms through 2.8 ohms, blue may indicate 2.8 ohms through 3 ohms, and purple may indicate 3 ohms through 3.2 ohms. Notably, the local view may provide biometric data of a second modality when compared to the global view which provides a larger or different portion of a body part with data from a first modality different than the second modality.

A medical professional may be able to view the visual data from the two modalities and ascertain more information than if only a single modality was provided. For example, a physician may be provided a cardiac chamber such that LAT values are rendered on the surface of the chamber. The physician may identify a specific area on the cardiac chamber that exhibits a potentially problematic characteristic. According to this example, LAT values alone may not allow the physician to discern enough information required to confirm if the specific area exhibits the problematic characteristic. Accordingly, as disclosed herein, a local view may enable the physician to see biometric data of an additional modality (e.g., a bipolar mapping or a dominant frequency) which may enable the physician to confirm that the specific area is a problematic area or is not a problematic area.

It will be understood that although the disclosure provided herein recites components, attributes, data, renders, and the like, as first, second, third, etc. (referred to as "items"), such designators are provided to distinguish between two or more items and are not necessarily provided to impose an order. As specific examples, a first portion of a body part is distinguished from a second portion of a body part such that the second portion of a body part is a subset of the first portion of a body part. As another example, a first set of biometric data may correspond to a first modality and a first portion of the body part. The first set of biometric data is distinguished from a second set of biometric data that may correspond to a second modality of a second portion of the body part.

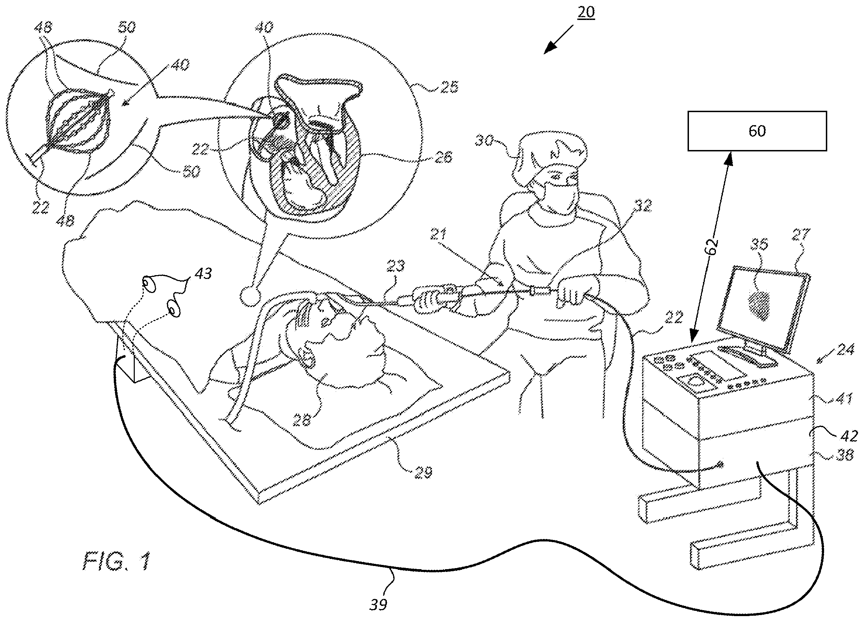

FIG. 1 is a diagram of an exemplary mapping system 20 in which one or more features of the disclosure subject matter can be implemented. Mapping system 20 may include a device, such as a catheter 40, that is configured to obtain biometric data in accordance with an embodiment of the disclosed subject matter. Although catheter 40 is shown to have a basket shape, it will be understood that a catheter of any shape that includes one or more elements (e.g., electrodes) may be used to implement the embodiments disclosed herein. Mapping system 20 includes a probe 21, having a shaft 22 that may be navigated by a medical professional 30 into a body part, such as heart 26, of a patient 28 lying on a table 29. As shown in FIG. 1, medical professional 30 may insert shaft 22 through a sheath 23, while manipulating the distal end of shaft 22 using a manipulator 32 near the proximal end of the catheter and/or deflection from the sheath 23. As shown in an inset 25, catheter 40 may be fitted at the distal end of shaft 22. Catheter 40 may be inserted through sheath 23 in a collapsed state and may be then expanded within heart 26.

According to an embodiment, catheter 40 may be configured to obtain biometric data of a cardiac chamber of heart 26. Inset 45 shows catheter 40 in an enlarged view, inside a cardiac chamber of heart 26. As shown, catheter 40 may include an array of elements (e.g., electrodes 48) coupled onto splines that form the shape of the catheter 40. The elements (e.g., electrodes 48) may be any elements configured to obtain biometric data and may be electrodes, transducers, or one or more other elements. It will be understood that although one catheter 40 is shown, multiple catheters may be used to collect biometric data of different modalities.

According to embodiments disclosed herein, biometric data may include one or more of LAT, electrical activity, topology, bipolar mapping, dominant frequency, impedance, or the like. The local activation time may be a point in time of a threshold activity corresponding to a local activation, calculated based on a normalized initial starting point. Electrical activity may be any applicable electrical signals that may be measured based on one or more thresholds and may be sensed and/or augmented based on signal to noise ratios and/or other filters. A topology may correspond to the physical structure of a body part or a portion of a body part and may correspond to changes in the physical structure relative to different parts of the body part or relative to different body parts. A dominant frequency may be a frequency or a range of frequencies that is prevalent at a portion of a body part and may be different in different portions of the same body part. For example, the dominant frequency of a pulmonary vein of a heart may be different than the dominant frequency of the right atrium of the same heart. Impedance may be the resistance measurement at a given area of a body part and may be calculated as a standalone value, based on a frequency, and/or in combination with additional considerations such as blood concentration.

As shown in FIG. 1, the probe 21 and catheter 40 may be connected to a console 24. Console 24 may include a processor 41, such as a general-purpose computer, with suitable front end and interface circuits 38 for transmitting and receiving signals to and from catheter 40, as well as for controlling the other components of mapping system 20. In some embodiments, processor 41 may be further configured to receive biometric data and generate rendering data for a global view and local view, based on the biometric data, as further disclosed herein. According to embodiments, the rendering data may be used to provide the medical professional 30 with a rendering of one or more body parts on a display 27, e.g., a body part rendering 35. According to an embodiment, the processor may be external to the console 24 and may be located, for example, in the catheter, in an external device, in a mobile device, in a cloud-based device, or may be a standalone processor.

As noted above, processor 41 may include a general-purpose computer, which may be programmed in software to carry out the functions described herein. The software may be downloaded to the general-purpose computer in electronic form, over a network, for example, or it may, alternatively or additionally, be provided and/or stored on non-transitory tangible media, such as magnetic, optical, or electronic memory. The example configuration shown in FIG. 1 may be modified to implement the embodiments disclosed herein. The disclosed embodiments may similarly be applied using other system components and settings. Additionally, mapping system 20 may include additional components, such as elements for sensing biometric patient data, wired or wireless connectors, processing and display devices, or the like.

According to an embodiment, a display connected to a processor (e.g., processor 41) may be located at a remote location such as a separate hospital or in separate healthcare provider networks. Additionally, the mapping system 20 may be part of a surgical system that is configured to obtain anatomical and electrical measurements of a patient's organ, such as a heart, and performing a cardiac ablation procedure. An example of such a surgical system is the Carto® system sold by Biosense Webster.

The mapping system 20 may also, and optionally, obtain biometric data such as anatomical measurements of the patient's heart using ultrasound, computed tomography (CT), magnetic resonance imaging (MRI) or other medical imaging techniques known in the art. The mapping system 20 may obtain electrical measurements using catheters, electrocardiograms (EKGs) or other sensors that measure electrical properties of the heart. The biometric data including anatomical and electrical measurements may then be stored in a local memory 42 of the mapping system 20, as shown in FIG. 1. Notably, memory 42 may store biometric data for multiple different modalities at the same time. The biometric data may be transmitted to the processor 41 from the memory 42. Alternatively, or in addition, the biometric data may be transmitted to a server 60, which may be local or remote, using a network 62.

Network 62 may be any network or system generally known in the art such as an intranet, a local area network (LAN), a wide area network (WAN), a metropolitan area network (MAN), a direct connection or series of connections, a cellular telephone network, or any other network or medium capable of facilitating communication between the mapping system 20 and the server 60. The network 62 may be wired, wireless or a combination thereof. Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology generally known in the art. Additionally, several networks may work alone or in communication with each other to facilitate communication in the network 62.

In some instances, the server 60 may be implemented as a physical server. In other instances, server 60 may be implemented as a virtual server a public cloud computing provider (e.g., Amazon Web Services (AWS) ®).

Control console 24 may be connected, by a cable 39, to body surface electrodes 43, which may include adhesive skin patches that are affixed to the patient 28. The processor, in conjunction with a current tracking module, may determine position coordinates of the catheter 40 inside the body part (e.g., heart 26) of a patient. The position coordinates may be based on impedances or electromagnetic fields measured between the electrodes 43 and the electrodes 48 or other electromagnetic components of the catheter 40.

Processor 41 may comprise real-time noise reduction circuitry typically configured as a field programmable gate array (FPGA), followed by an analog-to-digital (A/D) ECG (electrocardiograph) or EMG (electromyogram) signal conversion integrated circuit. The processor 41 may pass the signal from an A/D ECG or EMG circuit to another processor and/or can be programmed to perform one or more functions disclosed herein.

Control console 24 may also include an input/output (I/O) communications interface that enables the control console to transfer signals from, and/or transfer signals to electrodes 48 and electrodes 43. Based on signals received from electrodes 48 and/or electrodes 43, processor 41 may generate rendering data that enables a display, such as display 27 to render a body part, such as a body part rendering 35 and biometric data of multiple modalities as part of the body part rendering 35.

During a procedure, processor 41 may facilitate the presentation of a body part rendering 35, including a global view with a first modality and a local view with a second modality, to medical professional 30 on a display 27, and store data representing the body part rendering 35 in a memory 42. Memory 42 may comprise any suitable volatile and/or non-volatile memory, such as random-access memory or a hard disk drive. In some embodiments, medical professional 30 may be able to manipulate a body part rendering 35 using one or more input devices such as a touch pad, a mouse, a keyboard, a gesture recognition apparatus, or the like. In alternative embodiments, display 27 may include a touchscreen that can be configured to accept inputs from medical professional 30, in addition to presenting a body part rendering 35, including a global view and a local view.

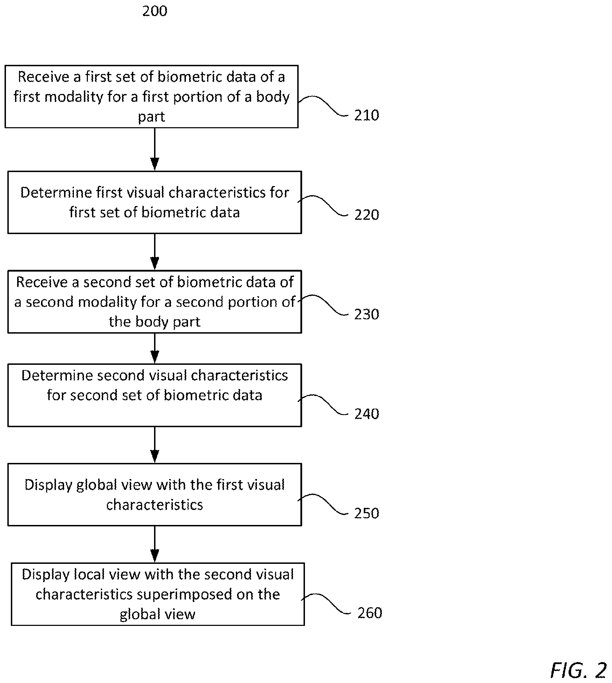

FIG. 2 shows a process 200 for displaying a global view with a first modality and a local view with a second modality, as disclosed herein. At step 210 of the process 200, a first set of biometric data of a first modality for a first portion of a body part may be received. The body part may be any body part or part of a body part in a patient's body such as an organ, muscle, tissue, ligament, or the like. For example, the body part may be a heart and the first portion of the body part may be a chamber in the heart or a part of a chamber in the heart. The first set of biometric data of the first modality may be sensed by a catheter, such as catheter 40 of FIG. 1. The catheter may include one or more elements, such as electrodes or transducers, that may be configured to sense the first set of biometric data. The catheter may be inserted into a patient's body through a natural orifice or through an incision created at a location on the patient's body. The catheter may traverse the surface of the body part (e.g., heart) and may collect the first set of biometric data during time intervals when the catheter rests at various points on the surface of the body part. As an example, the catheter may rest on 500 different points on the surface of a heart and collect LAT values over 2.5 second intervals during which intervals the catheter rest on each of the 500 different points on the surface of the heart. The first set of biometric data may be stored in a memory, such as memory 42 of FIG. 1.

The first set of biometric data of the first modality may be received by a processor, such as processor 41 of FIG. 1. The first set of biometric data of the first modality may be received by the processor over a wired or wireless connection between the catheter, such as catheter 40 of FIG. 1 and the processor, such as processor 41. The first set of biometric data of the first modality may be received by the processor as each data point is sensed by the catheter or the catheter may sense the entire first set of biometric data and provide it to the processor once the entire set is sensed.

A first range of values for the first set of biometric data of the first modality may be determined. The first range of values may be determined by a processor such as processor 41 of FIG. 1. The first range of values may be determined based on the maximum and minimum biometric data values within the first set of biometric data. For example, if the first set of biometric data contains LAT values as the first modality, such that the LAT values range from a lowest LAT value of -500ms to a highest LAT value of +500ms, then the first range may be -500ms to 500ms. The first range of values may be a filtered set of values where the filter may be, for example, a high pass filter, a low pass filter, an averaging filter, a filter that removes outlier values, or the like. For example, a filter may be applied such that 5% of the lowest values within the first set of biometric data and 5% of the highest values within the first set of biometric data are removed from the first set of biometric data. The first range of values may be stored in a memory, such as memory 42 of FIG. 1.

At step 220 of the process illustrated in FIG. 2, first visual characteristics corresponding to the values in the first set of biometric data of the first modality may be determined. The determination may be based on the first range of values. The first visual characteristics may be any visual characteristics that visually convey the different values in the first set of biometric data of the first modality and may be one or more of colors, hues, saturations, patterns, shapes, protrusions, textures, or alphanumeric characters. For example, the visual characteristics may be different colors that correspond to respective different ranges of LAT values. The values in the first set of biometric data may be segmented and a different visual characteristic from the first visual characteristics may be assigned to each segment. The number of values represented by each visual characteristic may be determined based on how wide or narrow the first range of values is. A wide range of values may result in segments that include a larger number of values in each segment and a narrow range of values may result in segments that include a smaller number of values in each segment. As described in the example provided herein, if the values in the first set of biometric data range from -500ms to +500ms, then the visual characteristics used to indicate the LAT values may be, for example, five colors including red, yellow, green, blue, purple such that red may indicate a segment of -500ms through -301ms, yellow may indicate a segment of -300ms through -101ms, green may indicate a segment of - 100ms through +99ms, blue may indicate a segment of + 100ms through +299ms, and purple may indicate a segment of +300ms through +500ms.

Notably, the first visual characteristics for the first set of biometric data of a first modality which corresponds to a first portion of a body part (e.g., the whole body part or a portion of the body part) may be used to display a global view of the body part over which a local view of a smaller subset of the body part (i.e., second portion of the body part) is superimposed and provides biometric data for a different modality than the global view, as further described herein.

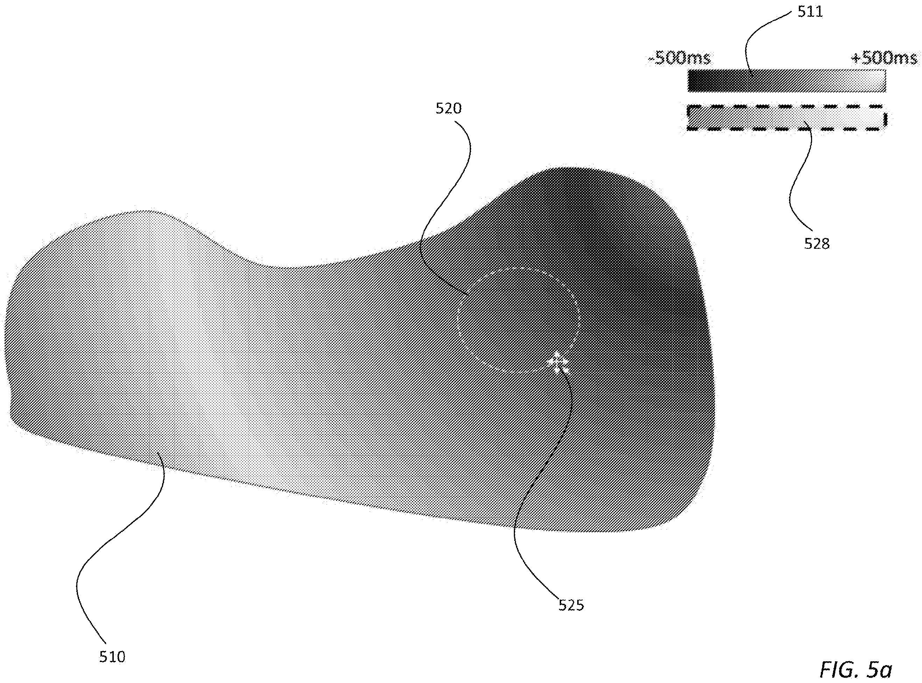

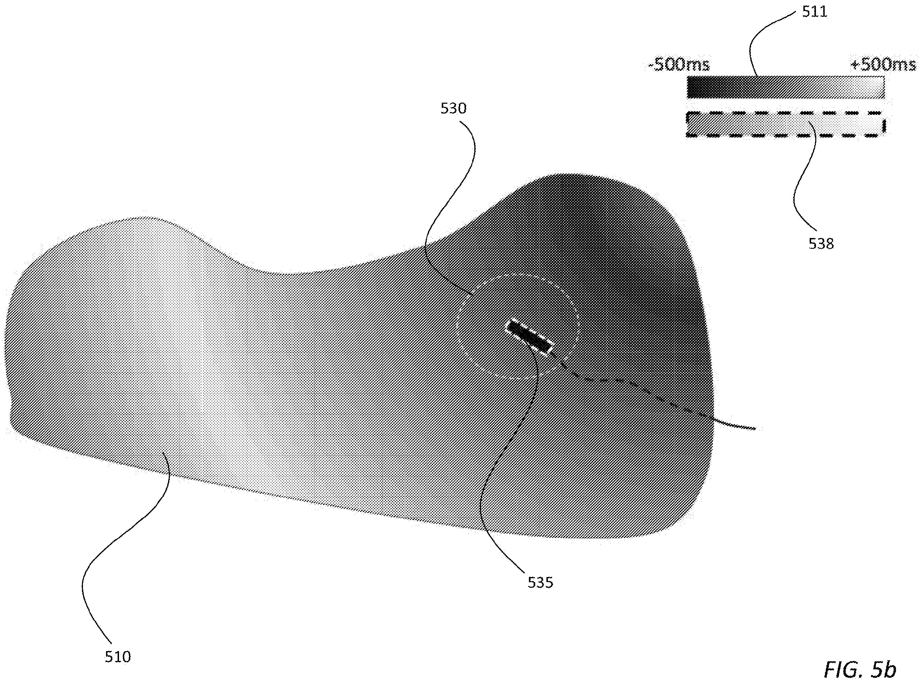

A second portion of the body part may be determined. The second portion of the body part may be a subset of the first portion of the body part such that the second portion of the body part corresponds to an area within the first portion of the body part. The second portion of the body part may be determined based on user input, as illustrated in FIG. 5A, as further descried herein. Alternatively, the second portion of the body part may be determined based on the location of a catheter, such as the catheter that is used to sense the first set of biometric data at step 210 of the process illustrated in FIG. 2, as illustrated in FIG. 5B, as further described herein.

At step 230 of the process illustrated in FIG. 2, a second set of biometric data of a second modality for a second portion of a body part may be received. The second portion of the body part may be a subset of the first portion of the body part such that the second portion of a body part is a smaller portion of a first portion of the body part. The second set of biometric data of the second modality may include biometric data values sensed by a different catheter that sensed the first biometric data or by the same catheter that sensed the first biometric data (e.g., by a different electrode, based on different electrode configuration, etc.). The catheter that senses the second biometric data of the second modality may be similar to or the same as catheter 40 of FIG. 1 and the second biometric of the second modality may be stored in a memory, such as memory 42.

A second range of values in the second set of biometric data may be determined. The second range of values may be determined by a processor such as processor 41 of FIG. 1. The second range of values may be determined based on the maximum and minimum biometric data values within the second set of biometric data of the second modality. For example, if the second set of biometric data includes impedance values that range from a lowest impedance value of 22 ohms to a highest impedance value of 32 ohms, then the range may be 22 ohms to 32 ohms. The second range of values may be a filtered set of values as described herein for the disclosure related to the first range of values.

The second modality corresponding to the second set of biometric data may be determined based on a system configuration, by user input, system resources, predetermined criteria, dynamically determined criteria, patient history, or the like. For example, the second modality may be determined based on a previous user selection of the modality. Alternatively, for example, the second modality may be determined based on a random selection of available second modalities and the second modality may be changed based on user input or the identification of a problem area, as further disclosed herein.

At step 240 of the process illustrated in FIG. 2, second visual characteristics corresponding to the values in the second set of biometric data of the second modality may be determined based on the second range of values. The second visual characteristics may be any visual characteristics that visually convey the different values in the second set of biometric data of the second modality and may be one or more of colors, hues, saturations, patterns, shapes, protrusions, textures, or alphanumeric characters. The second visual characteristics may be the same as or a subset of the visual characteristics determined based on the first range of values for the first subset of biometric data of the first modality. As an example of the second visual characteristics, the second visual characteristics may be different colors that correspond to respective ranges of impedance values. The values in the second set of biometric data of the second modality may be segmented and a different visual characteristic from the second visual characteristics may be assigned to each segment. The number of values represented by each visual characteristic may be determined based on how wide or narrow the second range of values is. As described in the example provided herein, if the values in the second set of biometric data of the second impedance based modality range from 22 ohms to 32 ohms, then the second visual characteristics used to indicate the impedance values may be the same five colors as the first visual characteristics, including red, yellow, green, blue, purple such that red may indicate a segment of 22 ohms to 24 ohms, yellow may indicate 24 ohms to 26 ohms, green may indicate 26 ohms to 28 ohms, blue may indicate 28 ohms to 30 ohms, and purple may indicate 30 ohms to 32 ohms.

Notably, the visual characteristics for the second set of biometric data of the second modality which corresponds to the second portion of a body part (i.e., a subset of the first portion of the body part) may be used to display a different category of data specific to the second portion of the body part when compared to the global view that includes first biometric data corresponding to the first modality of the larger first portion of the body part, as further described herein.

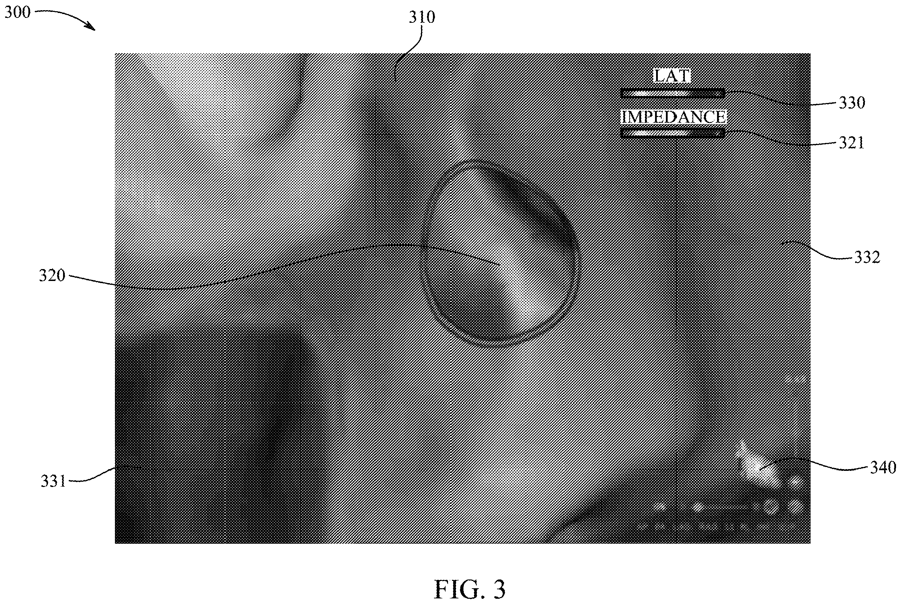

At step 250 of the process illustrated in FIG. 2, a global view with the first visual characteristics for the first modality may be displayed. The global view may include a render of the first portion of the body part such that the surface of the first portion of the body part is rendered using the first visual characteristics specific to the first modality. Accordingly, the global view may show the first portion of the body part with the first visual characteristics that visually indicate the values of the first set of biometric data of the first modality via the first visual characteristics. FIG. 3 is an image 300 of a display that shows a global view 310 of a first portion of a cardiac chamber. As shown in FIG. 3, the global view 310 is a render of a first portion of the cardiac chamber and the surface of the render of the first portion of the cardiac chamber is represented by light grey to dark grey visual characteristics which correspond to a LAT based modality. The light grey portions of the surface correspond to the lower LAT values as indicated in legend 330 and the dark grey portions of the surface corresponds to higher LAT values, as also indicated in the legend 330.

FIG. 3 also shows a reference orientation 340 that indicates the orientation of the cardiac chamber that is currently displayed via the global view 310. The orientation of the global view 310 may be changed and the reference orientation 340 may adjust based on the change. For example, a user may provide an input by pressing on a mouse button and moving the mouse to rotate the global view 310 such that a different area of the cardiac chamber is displayed. According to this example, the reference orientation 340 may change to reflect the change in orientation of the cardiac chamber being displayed.

At step 260 of the process illustrated in FIG. 2, a local view with the second visual characteristics of a second modality may be displayed. The local view may be superimposed on the global view and may include a render of the second portion of the body part such that the surface of the second portion of the body part is rendered using the second visual characteristics of the second modality. The second portion of the body part may be a subset of the first portion of the body part, as disclosed herein. Accordingly, the local view may show the second, smaller, portion of the body part with the visual characteristics that indicate the values of the second set of biometric data of a second modality. FIG. 3 shows a local view 320 of a second portion of a cardiac chamber superimposed on the global view 310. As shown in FIG. 3, the local view 320 includes a render of a second portion of the cardiac chamber and the surface of the render of the second portion of the cardiac chamber is represented by range of light grey to dark grey visual characteristics that correspond to changes in impedance values at the second portion of the body part. Similarly, the dark grey portions of the surface within the local view 320 correspond to higher impedance values within the smaller area occupied by the second portion of the cardiac chamber. Notably, the local view 320 of the second, smaller, portion of the cardiac chamber includes visual characteristics that correspond to a different category of biometric data (i.e., different modality) than the visual characteristics of the first, larger, portion of the cardiac chamber. Legend 321 shows the range of colors that correspond to a range of impedance values and is based on the local view 320.

Data for the global view, such as global view 310 of FIG. 3, and the local view, such as local view 320, may be generated by a processor, such as processor 41 of FIG. 1 and may be provided to a display, such as display 27. The processor may update the global view and/or the local view based on updated first set of biometric data for the first modality or second set of biometric data for the second modality and the global view and local view rendered on a display may be updated accordingly. The values of the updated first set of biometric data of the first modality or second set of biometric data of the second modality may be provided based on additional biometric data sensed by a catheter, such as catheter 40.

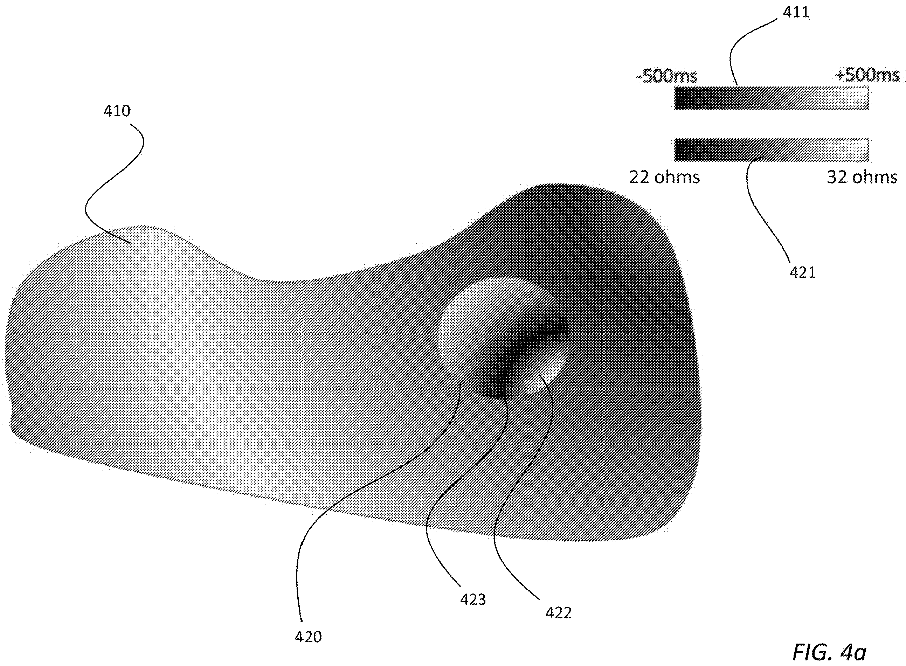

FIG. 4A shows a simplified illustration of a global view 410 and local view 420, in accordance with embodiments disclosed herein. As shown in FIG. 4A, global view 410 includes a render of a first portion of a body part using first visual characteristics that include gradients of grey that range from light grey to dark grey. The first visual characteristics of the global view 410 correspond to a range of LAT values that range from -500ms to +500ms as shown in global view legend 411. Notably, the global view 410 of the first, larger, portion of the body part is rendered using first visual characteristics that correspond to a LAT value-based modality when compared to the local view 420 which is rendered using visual characteristics that correspond to an impedance value-based modality. The local view 420, which corresponds to a smaller second portion of the body part, that is a subset of the first portion of the body part, includes a render of the smaller second portion of the body part using second visual characteristics that include gradients of grey that range from light grey to dark grey. The second visual characteristics of the local view 420 correspond to impedance values that range from 22 ohms to 32 ohms as shown in local view legend 421. As shown in the local view 420, the dark grey visual characteristic 423 and light grey visual characteristic 422 correspond to granular differences in impedance (i.e., second modality) based biometric data, within the area occupied by the second portion of the body part.

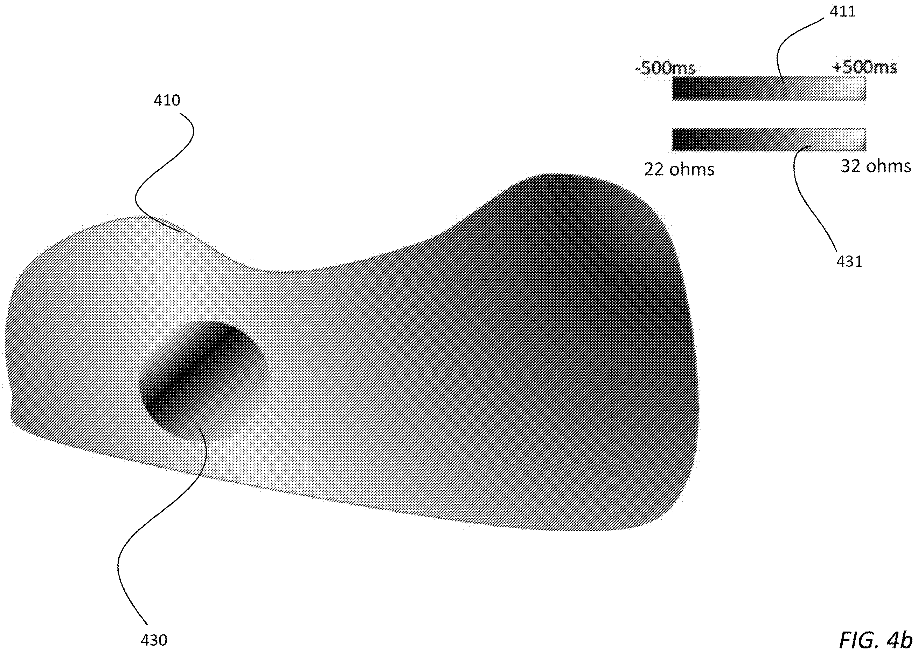

According to an embodiment of the disclosed subject matter, as shown in FIG. 4B, a different local view 430 may be shown superimposed on the same global view 410 as shown in FIG. 4A. The local view 430 may correspond to a smaller body part that is a subset of the first body part that corresponds to the global view 410. The local view 430 may be rendered in response to receiving a selection of the smaller body part that corresponds to the location of the local view 430. The selection of the smaller body part may be provided by a user, as shown in FIG. 5A, as further disclosed herein. Alternatively, the local view 430 may be rendered in response to the movement of a catheter to the area that corresponds to the location of the local view 430, as shown in FIG. 5B and further disclosed herein.

The local view 430 of FIG. 4B may include visual characteristics that are determined based on the second modality range of biometric data values specific to the second portion of the body part associated with the area of the local view 430, which is different than the modality shown in global view 410 (i.e., LAT values). The modality for local view 430 may be different from or the same as the modality for local view 420 of FIG. 4A.

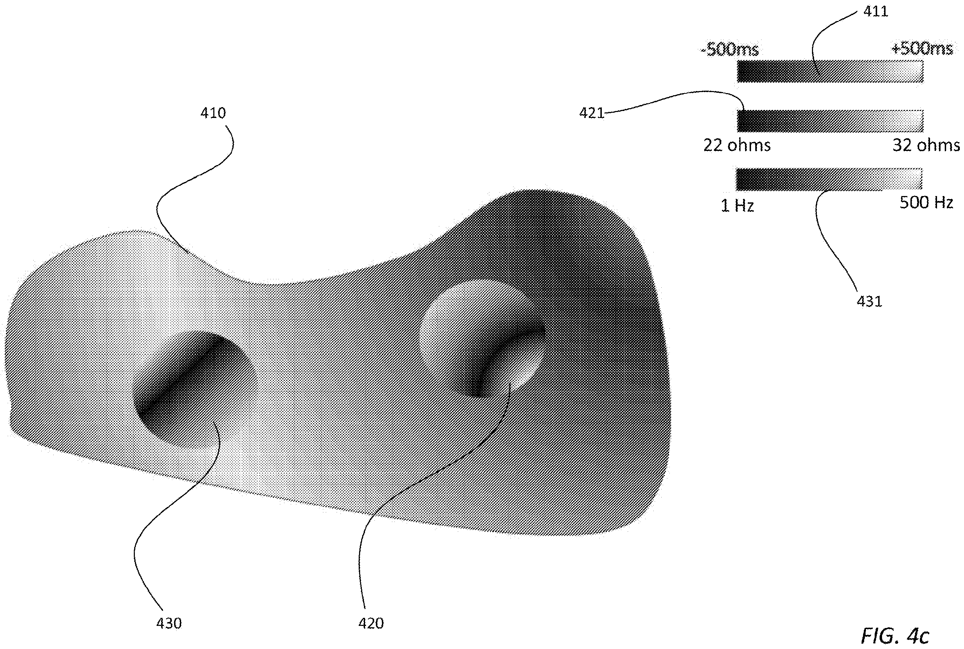

According to an embodiment of the disclosed subject matter, as shown in FIG. 4C, two or more local views, such as local view 420 and local view 430 may be displayed simultaneously. The number of local views to be displayed simultaneously may be determined by a system configuration, by user input, system resources, predetermined criteria, dynamically determined criteria, or the like. As shown in FIG. 4C, local views 420 and 430 may be superimposed onto the global view 410 simultaneously. The modality for local view 420 may be the same as the modality for local view 430 (not shown). Alternatively, as shown, the modality for local view 420 may be different than the modality for local view 430. As indicated by the local view legend 421, the modality for the local view 420 may be impedance and the modality for the local view 430 may be a dominant frequency. The values for the impendence values shown in local view 420 may range from 22 ohms to 32 ohms and the values for the dominant frequency values shown in local view 430 may range from 1 Hz to 500 Hz.

FIG. 5A shows a simplified illustration of a user selection 520 of a second portion of a body part. As shown in FIG. 5A, a global view 510 may be rendered and may convey biometric data information using visual characteristics determined based on the range of values of the biometric data of a first modality associated with a first portion of a body part, the range shown via global view legend 511. A user may provide an input for the selection of a second, smaller, portion of a body part that is a subset of the first portion of the body part that corresponds to the global view 510. The user input may be provided any applicable means such as by a physical input device (e.g., mouse, keyboard, a touch screen, stylus, etc.), a voice command, a gesture, or the like. FIG. 5A shows an example of a circular user selection that may be provided by a user depressing a mouse button when cursor 525 is towards a top portion of the circle that makes up the user selection 520 and dragging the mouse down towards the position of the cursor 525 shown in FIG. 5A. The user may release the mouse button and the user selection 520 may be treated as an input that determines the second body portion corresponding to the user selection 520. The second body portion corresponding to the user selection 520 may be applied, at step 230 of the process disclosed in FIG. 2, to determine a second range of values of a second modality in a second set of biometric data corresponding to the second portion of the body part based on the user selection 520. The range of biometric data corresponding to the second portion of the body part may be shown in local view legend 528 which may change based on the area selected by the user and the given modality for the area selected by the user.

FIG. 5B shows a simplified illustration of a determination of a second portion of a body part based on the location of catheter 535. As shown in FIG. 5B, a global view 510 may be rendered and may convey biometric data information of a first modality using visual characteristics determined based on the range of values of the biometric data associated with a first portion of a body part, the range shown via global view legend 511. The location of a catheter 535 may be determined based on visual cues, electromagnetic transmissions that facilitate communication with a location track pad or electrodes 39 of FIG. 1. The location of the catheter 535 may result in a determination of the second portion of the body part corresponding to the selected area 530. The second portion of the body part corresponding to the selected area 530 may be applied, at step 230 of the process disclosed in FIG. 2, to determine a second range of values of a second modality in a second set of biometric data corresponding to the second body portion based on the selected area 530. Notably, the location of the selected area 530 may change based on movement of the catheter 535. The range of biometric data of a second modality corresponding to the second portion of the body part may be shown in local view legend 538 which may change based on the location of the catheter 535.

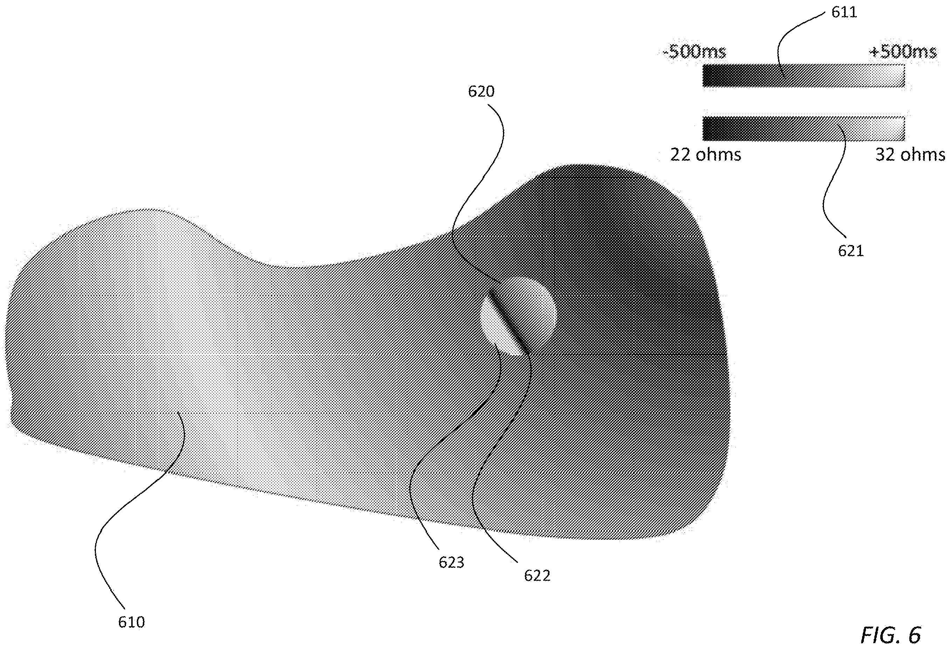

According to an embodiment of the disclosed subject matter, a local area may enable identification of problem areas such as scars, dead tissue, overly active tissue, electric signal rotors, or the like. FIG. 6 shows a global view 610 that may be rendered and may convey biometric data information using visual characteristics determined based on the range of values of the biometric data of the first modality associated with a first portion of a body part, the range shown via global view legend 611. A local view 620 may be superimposed over the global view 610 and the local view 620 may correspond to a second portion of the body part for which the biometric data of a second condition exhibits a problem condition. The modality for the local view 620 may be determined based on the type of problem condition such that the modality may be selected such that it most prominently shows the problem area. For example, the global view 610 may show LAT values and a problem area may be identified which has large fluctuations in impedance. Accordingly, the second modality shown via a local view 620 may be impedance such that the large fluctuations in impedance are shown superimposed on the LAT values shown in the global view 610. Such a second portion of the body part may be identified based on an analysis of one or more modalities of biometric data corresponding to the entire first portion of the body part. The analysis may include evaluating changes in biometric data over an area, such as the surface area of a heart. Alternatively, or in addition, the analysis may include applying a predetermined filter or machine learning algorithm to the biometric data, of multiple modalities, for the first portion of the body part corresponding to the global view 610.

The second portion of the body part that corresponds to the local view 620 may be automatically identified based on the analysis of the biometric data of one or more modalities, corresponding to the entire first portion of the body part. Based on the automatic identification, the local view 620, with the corresponding modality, may be selected. The analysis may be conducted based on stored algorithms (e.g., in memory 42 of FIG. 1) that are generated based on previous or known identifications of problem areas.

As noted, a problem area may be one or more scars, dead tissue, overly active tissue, electric signal rotors, or the like. As an example, a scar may be identified by abnormal bipolar mapping values within a given area of a body part. The abnormal bipolar mapping values may, for example, be a sharp change in impedance values such as between impedance values 623 and 622 which correspond to the local view legend 621. The scar may not be visible when viewing the only the LAT values shown via the global view 610. However, the system, such as mapping system 20 of FIG. 1, may sense biometric data for multiple modalities and, based on the sensed biometric data for the multiple modalities, may identify both the problem area and the modality that most proximately identifies the problem area.

Another problem area may be a live tissue area between a scar. Such an area may be identified based on biometric data that includes bipolar amplitudes. The bipolar amplitudes for an entire first portion of a body part may span a wide range such that a live tissue area between a scar may not be visible on a global view. Similarly, another problem area may be a rotor signal, such as an electrical signal at a portion of a chamber that exhibits a circular pattern. A rotor signal is often indicative of a source of AFib. The biometric data of a first portion of a body part may include electrical activity which may be analyzed to detect a rotor signal. The electrical activity of the entire first portion of the body part may span a wide range such that the rotor signal may not be visible on a global view.

A local view, such as local view 620 of FIG. 6 may be provided over a global view 610 and may correspond to the area of the first portion of a body part that exhibits live tissue within scar tissue. The local view may correspond to a second, smaller, portion of the body part and may be a subset of the entire first portion of the body part rendered in the global view 610. The local view 620 may be rendered and may convey biometric data (e.g., the bipolar data to identify live tissue in a scar or the rotor signal based on electrical activity data) for a modality that most clearly visually displays the biometric data that indicates the presence of the problem area.

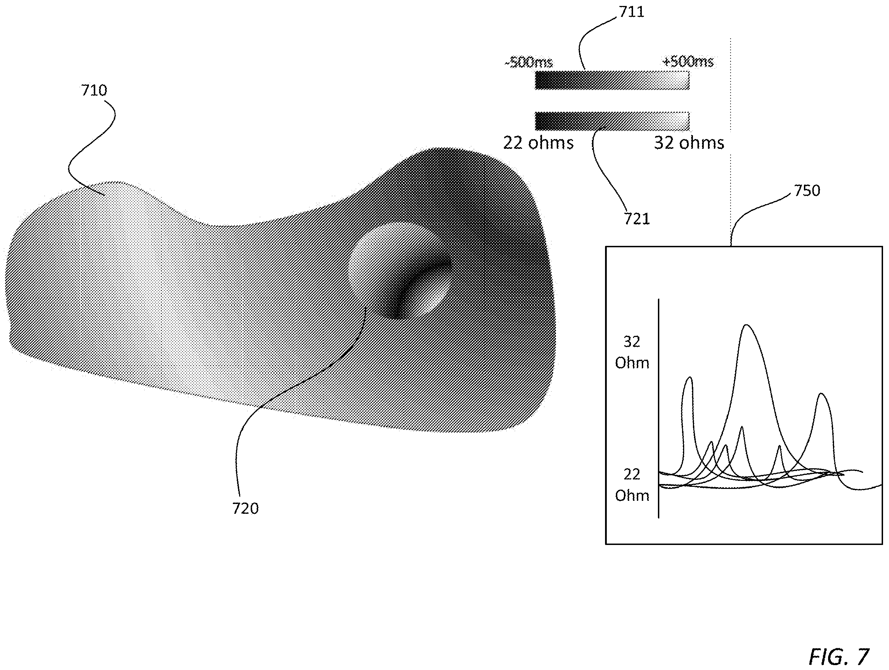

According to an embodiment of the disclosed subject matter, a third view may be provided external to a global view and may show details of the biometric data that corresponds to the second modality displayed via a local view. FIG. 7 shows an example third view 750 that shows the impedance values for various points within the local view 720 that the visual characteristics of the local view 720 are based on. As show, the visual characteristics for the global view 710 correspond to LAT values which correspond to global view legend 711 and the visual characteristics for the local view 720 correspond to impedance values which correspond to the local view legend 721. Additionally, the third view 750 includes values that range from 22 ohms to 32 ohms, which correspond to the range in the local view legend 721.

Any of the functions and methods described herein can be implemented in a general-purpose computer, a processor, or a processor core. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer-readable media). The results of such processing can be maskworks that are then used in a semiconductor manufacturing process to manufacture a processor which implements features of the disclosure.

Any of the functions and methods described herein can be implemented in a computer program, software, or firmware incorporated in a non-transitory computer-readable storage medium for execution by a general-purpose computer or a processor. Examples of non-transitory computer-readable storage mediums include a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs).

It should be understood that many variations are possible based on the disclosure herein. Although features and elements are described above in particular combinations, each feature or element can be used alone without the other features and elements or in various combinations with or without other features and elements.

## Claims

1. A method comprising:
receiving a first set of biometric data of a first modality for a first portion of a body part;
determining first visual characteristics based on the first set of biometric data;
receiving a second set of biometric data of a second modality for a second portion of the body part, the second portion of the body part being a subset of the first portion of the body part;
determining second visual characteristics based on the second set of biometric data; and
providing, for display, a first view comprising the first portion of the body part rendered with the first visual characteristics; and
providing, for display, a second view comprising the second portion of the body part rendered with the second visual characteristics, the second view superimposed on the first view at a location on the first view corresponding to the second portion of the body part.

2. The method of claim 1, further comprising providing, for display, a third view comprising a rendering based on the second set of biometric data.

3. The method of claim 1, wherein the body part comprises a cardiac chamber.

4. The method of claim 1, wherein the first visual characteristics are based on a range of values within the first set of biometric data and the second visual characteristics are based on a range of values within the second set of biometric data.

5. The method of claim 1, wherein the first modality or the second modality are determined based on one of an automatic determination or a user input.

6. The method of claim 5, wherein the automatic determination is based on one of a, system configuration, prior use, the first biometric data, the second biometric data, a patient history, and a stored setting.

7. The method of claim 1, further comprising:
receiving a third set of biometric data of a third modality for the second portion of the body part;
determining third visual characteristics based on the third set of biometric data; and
providing, for display, a third view comprising the second portion of the body part rendered with the third visual characteristics.

8. The method of claim 7, wherein the third view comprising the second portion of the body part rendered with the third visual characteristics is provided after the second view comprising the second portion of the body part rendered with the second visual characteristics is provided.

9. The method of claim 1, wherein the first portion of the body part is one of an entire body part or a subset of the body part.

10. A system comprising:
one or more catheters configured to sense a first set of biometric data of a first modality for a first portion of a body part and a second set of biometric data of a second modality for a second portion of the body part, the second portion of the body part being a subset of the first portion of the body part;
a processor configured to:
determine first visual characteristics based on the first set of biometric data;
determine second visual characteristics based on the second set of biometric data; and
a display configured to:
display a first view comprising the first portion of the body part rendered with the first visual characteristics; and
display a second view comprising the second portion of the body part rendered with the second visual characteristics, the second view superimposed on the first view at a location on the first view corresponding to the second portion of the body part.

11. The method of claim 1 or the system of claim 10, wherein the first modality is one of a local activation time (LAT), an electrical activity, a topology, a bipolar mapping, a dominant frequency, or an impedance and the second modality is a different one of a LAT, an electrical activity, a topology, a bipolar mapping, a dominant frequency, or an impedance, then the first modality.

12. The system of claim 10, wherein the first visual characteristics and the second visual characteristics are one or more of colors, hues, saturations, patterns, protrusions, textures, and alphanumeric characters.

13. The system of claim 10, wherein the one or more catheters are configured to sense the first biometric data and the second biometric data from within a patient's body.

14. The system of claim 10, wherein the first portion of the body part is one of an entire body part and a subset of the body part.

15. The system of claim 10, further comprising an input device configured to receive an indication of the second body part.

16. The system of claim 10, wherein the display is remotely located from the processor and the display is configured to communicate with the processor over a network.

17. A processor configured to:
receive a first set of biometric data of a first modality for a first portion of a body part;
receive a second set of biometric data of a second modality for a second portion of the body part, the second portion of the body part being a subset of the first portion of the body part;
provide, for display, a first view comprising the first portion of the body part rendered with first visual characteristics; and
provide, for display, a second view comprising the second portion of the body part rendered with second visual characteristics, the second view superimposed on the first view at a location on the first view corresponding to the second portion of the body part.
